# EUROPEAN PATENT APPLICATION

(11) **EP 3 888 599 A1**
(43) Date of publication of application: **06.10.2021**
(21) Application number: 21170602.3
(22) Date of filing: 24.11.2009
(51) Int. Cl.: A61F 2/38

(54) **KNEE JOINT FOAM CUSHION**

(30) Priority: 24.11.2008 US 193396 P
(62) Divisional of application: 09827813.8
(71) Applicant: Implantica Patent Ltd., 223 70 Lund (SE)
(72) Inventor: Forsell, Peter, 6300 Zug (CH)
(74) Representative: AWA Sweden AB

(57) **Abstract**

A cushion (30) is disclosed for insertion in a patient's knee joint to serve as a buffer between a patient's femur and tibia. The knee joint cushion is made from a flexible material (34) that allows relative movement between the patient's femur and tibia, while resisting rupture as it supports these two bones as they are moving relative to one another as the knee joint is flexed, bent or extended.

## Description

The present application relates to knee joint repairs, and more particularly, to a cushion for insertion in a patient's knee joint that acts as a buffer between the patient's upper and lower knee bones.

### BACKGROUND OF THE INVENTION

Most people experience some kind of knee problem at some time during their life. Many knee injuries occur during physically stressful activities, such as sports. It is possible, however, for knee problems to develop from everyday wear and tear or overuse of a person's knees.

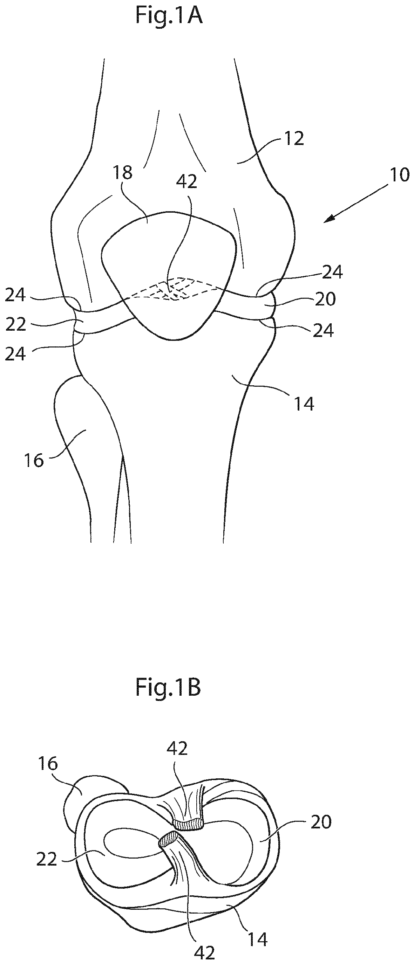

FIGURE 1A is a front view of a healthy knee joint 10. The knee joint 10 includes an upper bone called the femur or "thigh bone" 12, two lower bones called the tibia or "shin bone" 14 and the fibula 16, and a patella or "knee cap" 18. The upper and lower bones 12 and 14 are separated by two rubbery cushions or discs which are called the "medial meniscus" 20 and the "lateral meniscus" 22, which are best seen in the top view of FIGURE 1B. The upper and lower bones are also connected by ligaments, tendons and muscles (not shown). The surfaces of the upper and lower bones inside the knee joint 10 are covered by a layer of smooth, shiny cartilage called "articular cartilage" 24, which protects these bones, absorbs shock and provides a smooth, gliding surface for near frictionless movement of the bones as the knee joint 10 is flexed, bent or extended.

Sudden or acute injuries are the most common cause of knee problems. One kind of acute injury is one or more tears occurring in one or both of the menisci 20 and 22 that serve to cushion the knee joint 10.

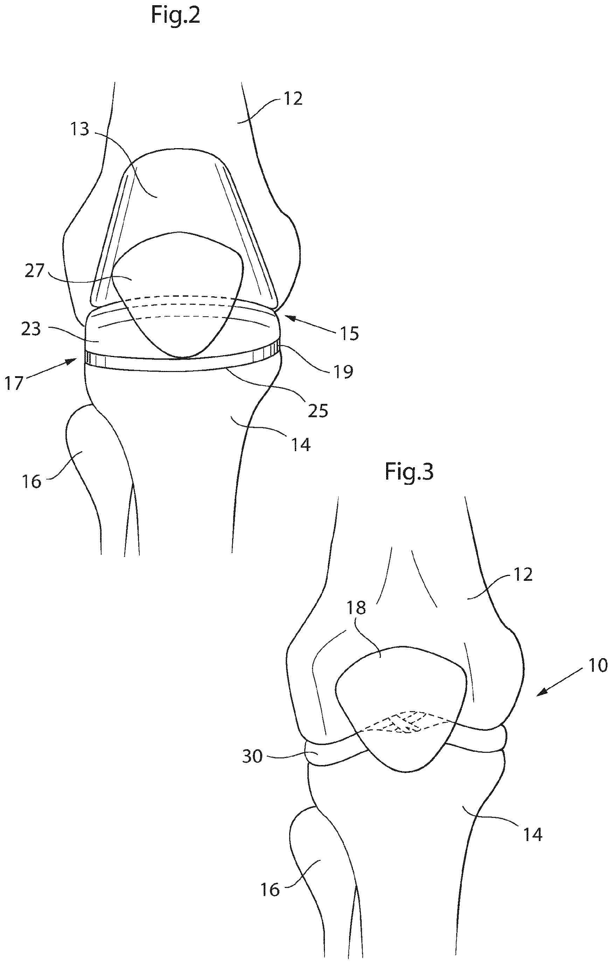

When a person's problems with a knee joint become severe enough, he or she may require a total knee replacement. FIGURE 2 is a front view of a conventional total knee replacement, or "arthroplasty" 11 in which the end surfaces of the upper and lower knee bones 12 and 14 are relined with artificial parts or "prostheses". Typically, there are three components used in a conventional knee replacement. The femoral (thigh) component 13 is made of metal and is inserted into the end 15 of the femur 12. The tibia (shin bone) component 17, is made of a metal piece 19 that covers the end 21 of the tibia 14 and a polyethylene (medical-grade plastic) piece 23, which together cover the top end 25 of the tibia 14. The metal piece 19 forms the base of the tibia component 17, while the polyethylene piece 23 is attached to the top of the metal piece 19 to serve as a cushion and smooth gliding surface for the metal femoral component 13. The third component, the patella (kneecap) 27 may be all polyethylene or a combination of metal and polyethylene. These new knee replacement components are then stabilized by a patient's ligaments and muscles, just as in a healthy knee.

One draw back to the conventional knee replacement shown in FIGURE 2 is the wear and tear in the knee replacement that can result from the metal femoral component 13 rubbing against the polyethylene piece 23 of the tibia component 17. As such, it would be desirable to provide a solution to knee joint injuries, like tears in one or both of the menisci 20 and 22 that cushion the upper and lower knee bones, that eliminates the wear and tear in a conventional knee replacement resulting from the metal femoral component 13 rubbing on the polyethylene piece 23 of the tibia component 17.

### BRIEF DESCRIPTION OF THE INVENTION

The present invention is directed to a cushion for insertion in a patient's knee joint to serve as a buffer between a patient's femur and tibia. The cushion is filled with a substantially incompressible fluid, which can be a foam or a gel or a liquid, like water, which is capable of supporting the entire weight of the patient's body. The cushion is preferably made from a flexible material that allows relative movement between the patient's femur and tibia, while resisting rupture as it supports these two bones as they are moving relative to one another as the knee joint is flexed, bent or extended. Preferably, the cushion is made from a polymer type of material.

The cushion has a single interior pocket or compartment which is filled by the substantially incompressible fluid. The cushion can also be formed with a plurality of individual interior pockets or compartments within the overall cushion pocket which are filled by the substantially incompressible fluid. The plurality of pockets or compartments allows the integrity of the cushion to remain largely intact so that the cushion can continue to buffer the upper and lower bones of a patient's knee, even where one of the cushion's compartments ruptures for one reason or another. The plurality of pockets or compartments also provides, if there is extra pressure in one area, the ability to move fluid away from that area.

The cushion can also include an opening or hole in its center to accommodate the two interior crucible ligaments extending between the bottom of the femur and the top of the tibia. Alternatively, the cushion can be shaped like a bow tie with a narrow center portion that can fit between the two interior crucible ligaments extending between the femur and tibia. Any other suitable shape may be used, depending on the situation in a patient's knee, such as for example where there are no crucial ligaments existing at all in a patient's knee.

A top side of the cushion is attached by suitable means to the bottom of the femur, while the bottom side of the cushion is attached to the top side of the tibia, so that as the femur moves relative to the tibia, when a patient bends his or her knee joint, the positioning of the points of attachment of the cushion to the femur and the tibia are caused to shift relative to one another to accommodate the change in orientation between the femur and the tibia caused by the bending of the patient's knee joint. This shift in the relative positioning of the attachment points eliminates the sliding, and, thus, wear and tear, that occurs between the components used in conventional knee replacements where an upper metal piece of the artificial knee joint slides relative to a lower plastic piece of the joint as the joint is flexed.

The cushion of the present invention can also include a valve arrangement that allows pressure to be added to or removed from the fluid filling the cushion to thereby change the level of support of a patient's body weight provided by the cushion. Where the cushion includes such a valve arrangement, a suitable pump and reservoir of fluid are implanted in the patient's leg to allow the fluid in the cushion to be adjusted and thereby change the pressure of the fluid within the cushion. Alternatively, the cushion could simply be filled using an implanted injection port.

In an alternative embodiment, two cushions can be inserted between the patient's femur and tibia. In this arrangement, the construction of each of the two cushions would be similar to that described above. However, the upper cushion would be attached on its upper side to the patient's femur, while the other, lower cushion would be attached on its lower side to the patient's tibia. Positioned between the two cushions are a pair of flat metal plates that, in one embodiment, would slide relative to one another as a patient would flex his or her knee so that the orientation of the femur relative to the tibia would change. In this arrangement, the upper cushion would be attached on its lower side to the upper metal plate, while the lower cushion would be attached on its upper side to the lower metal plate. In a further alternative embodiment, the two metal plates would be fixed relative to one another so that flexing of a patient's joint would result in shifting of the fluid within each of the two cushions so as to accommodate a change in the orientation of the patient's femur relative to the patient's tibia as the patient's knee joint is flexed.

In all of the foregoing embodiments, the cushion (s) can be inserted in a knee joint, either after the patient's menisci have been removed due to injury, or with the patient's menisci still in place, but diminished in their dimensions due to wear and tear on them.

In yet another embodiment, the flexible pocket is adapted to at least partly be attached to the tibia and femoral bones, wherein the flexible fluid filled pocket is adapted to allow a friction free movement between the tibia and femur. The movement is instead based on material flexibility and fluid movement

The cushion could also have a first fixating section for attaching the cushion to the bottom of the femur at, at least a first point of attachment and a second fixating section for attaching the cushion to the top of the tibia at at least a second point of attachment. As the femur moves relative to the tibia when a patient bends his or her knee joint, the positioning of the first and second points of attachment are caused to shift relative to one another to accommodate a change in orientation between the femur and the tibia caused by the bending of the patient's knee joint.

According to yet another embodiment, the flexible pocket in both the first and second cushion is adapted to at least partly be attached to the tibia and femoral bones. The flexible fluid filled pocket is adapted to allow friction free movement between the tibia and femur. The movement is instead based on material flexibility and fluid movement. The cushion could further comprise a first and second flat plate, positioned between the cushion and the second cushion, the first and second plates being attached to the cushion and the second cushion so that the plates slide relative to one another as the orientation between the femur and the tibia changes as the patient bends his or her knee, wherein said plates is adapted to be placed parallel to each other for allowing a larger weight carrying surface.

A method for implanting a cushion in a patient's knee joint is further provided, the method comprises the steps of: placing a pocket made from a flexible material, with a fluid filling the interior of the pocket fitting in the space between the patient's femur and tibia bones.

### BRIEF DESCRIPTION OF THE DRAWINGS

FIGURE 1A is a front elevational view of a healthy knee joint.
FIGURES 1B is a top view of the medial meniscus and the lateral meniscus separating and cushioning the upper and lower knee joint bones.
FIGURE 2 is a front view of a conventional total knee replacement.
FIGURE 3 is a front view of a knee joint in an unflexed position with the cushion of the present invention implanted between a patient's femur and tibia.
FIGURES 4A to 4E are top views of several embodiments of the cushion of the present invention.

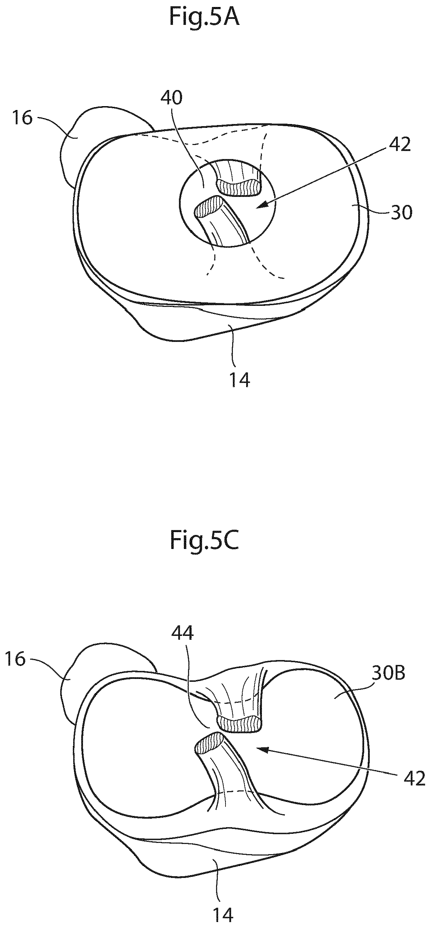

FIGURES 5A and 5B are top views of two of the embodiments of the cushion of the present invention showing their positioning on the top of a patient's tibia depicting how the cushion interacts with the two interior crucible ligaments extending from the top of the tibia to the bottom of the femur.
FIGURE 6A is a side view of a knee joint in an unflexed position with the cushion of the present invention implanted between a patient's femur and tibia.
FIGURE 6B is a side view of a knee joint in a flexed position with the cushion of the present invention implanted between a patient's femur and tibia.
FIGURE 7A and 7B is a side view of a knee joint in an unflexed/flexed position with two of the cushions of the present invention implanted between a patient's femur and tibia.
FIGURE 8 depicts one embodiment of an apparatus for performing the function of adding fluid to or removing fluid from the cushion of the present invention to thereby change the pressure in cushion
FIGURE 9 shows one embodiment of an injection port for adding fluid to or removing fluid from the cushion of the present invention.
FIGURES 10 and 11 show injection port of FIGURE 9 working as a hand driven pump.

### DETAILED DESCRIPTION OF THE INVENTION

FIGURE 3 is a front schematic view of a knee joint 10 in an unflexed position with the cushion 30 of the present invention implanted between a patient's femur 14 and tibia 12. As shown in FIGURE 3, the cushion 30 of the present invention is intended to be inserted in a patient's knee joint 10 to serve as a buffer between the patient's femur 14 and tibia 12, not unlike the menisci originally separating and cushioning the patient's femur 14 and tibia 12. As such, preferably, the cushion 30 will be sized to fit into a patient's knee joint like the original menisci 20 and 22 shown in FIGURE 1B. Preferably, the cushion 30 is filled with an substantially incompressible fluid 32, such as a foam or gel or liquid, like water, for example, which is capable of supporting the weight of a patient's body. The cushion 30 is preferably made from a flexible material 34 that allows relative movement between the patient's femur 14 and tibia 12, while supporting the weight of the patient, and while resisting rupture of the cushion 30 as it buffers the femur and tibia while they are moving relative to one another. Preferably, the cushion 30 is made from a polymer type of material. Preferably the cushion 30 is coated for increased fatigue resistance.

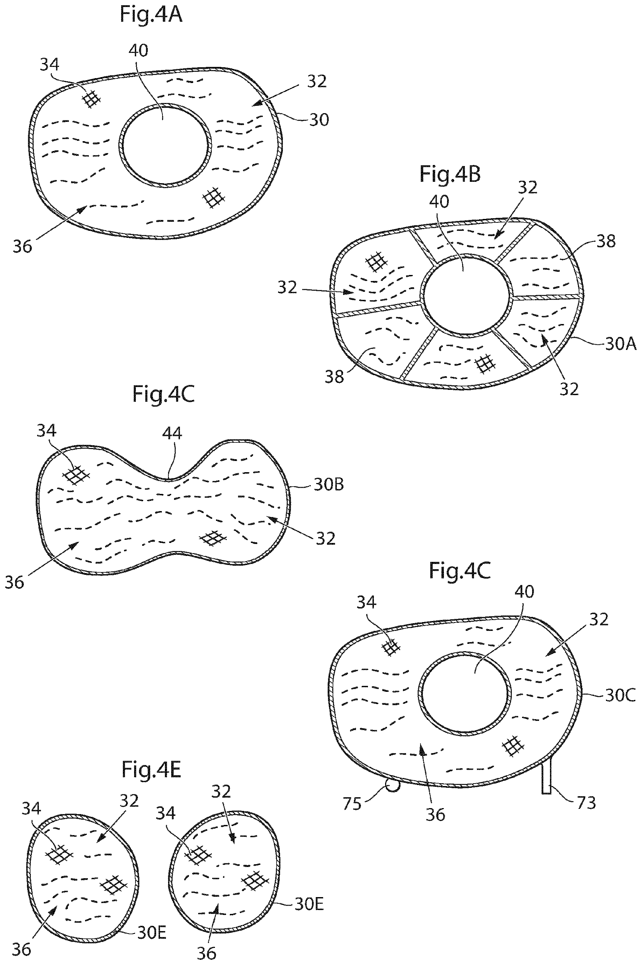

As shown in FIGURE 4A, the cushion can be formed with a single compartment 36 within which is located the substantially incompressible fluid 32. One difficulty with this design is the possibility of a complete failure of the cushion 30 where the flexible material 34 fails for some reason so that the single compartment 36 is completely ruptured, thereby allowing the fluid 32 in the compartment 36 to completely drain out of cushion 30.

FIGURE 4B is a top view of another embodiment 30A of the cushion of the present invention containing a plurality of compartments 38 within which the substantially incompressible fluid 32 is located. The cushion embodiment 30A shown in FIGURE 4B overcomes the rupturing problem of the cushion 30 of FIGURE 4A, to a degree, in that the plurality of compartments 38 within which the substantially incompressible fluid 32 is stored can prevent a complete failure of cushion 30A, if the flexible material 34 fails. In this design, if the cushion 30A is ruptured, it is likely that only a single one of the plurality of compartments 38 will rupture so as to lose the fluid 32 located in that compartment. The remaining un-ruptured compartments 38 comprising the cushion 30A will then be able to continue supporting the patient's weight and femur 14 as it is moved relative to the tibia 12 as the patient flexes his or her knee. Furthermore the compartments prevents unwanted movements of the fluid.

As shown in FIGURES 4A and 4B, each of the cushions 30 and 30A includes an opening or hole 40 in its center to accommodate the interior crucible ligaments 42 extending between the center of the bottom of the femur 14 and the top of the tibia 12. An alternative to this arrangement is the embodiment 30B of the cushion shown in FIGURE 4C. In the embodiment of FIGURE 4C, the cushion 30B is shaped like a bow tie with a narrow center portion 44 that can fit between the two interior crucible ligaments 42 extending between the femur 14 and tibia 12. It should be noted that the cushion 30B shown in FIGURE 4C could be made with plurality of compartments 38 within which the substantially incompressible fluid 32 is stored, as shown in FIGURE 4B, to again prevent a complete failure of cushion 30B where the flexible material 34 fails so as to cause only one of the plurality of compartments 38 to rupture.

The knee joint comprises a prolonged femoral length axis, being the prolongation of the length axis of the femoral bone which passes the knee joint and continues substantially to the tibia length axis, when the leg is in its fully extended state.

FIGURE 4E shows an implantable system comprising a first and second cushion according to any of the embodiments herein, the first cushion is adapted to mainly be positioned on the medial side of the femoral length axis, and the second cushion is adapted to mainly be positioned on the lateral side of the femoral length axis, such that the first cushion is adapted to mainly carry a load originating from the weight of the patient distributed through the medial condyle, and the second cushion is adapted to mainly carry a load originating from the weight of the patient distributed through the lateral condyle, when the first and second cushions are implanted.

According to another embodiment (not shown), a system could further comprise a third cushion positioned between the first cushion placed at the medial condyle and either at the femur or the tibia bones so that two cushions are positioned between the bones. The first cushion has a first fixating section for attaching the cushion to the bottom of the femur at, at least a first point of attachment, and the third cushion has a third fixating section for attaching the third cushion to the top of the tibia at, at least a third point of attachment. The system could further comprise a fourth cushion positioned between the second cushion and either the femur or the tibia bones at the lateral condyle, so that two cushions are positioned between the bones. The second cushion has a second fixating section for attaching the cushion to the bottom of the femur at, at least a second point of attachment and the fourth cushion has a fourth fixating section for attaching the fourth cushion to the top of the tibia at, at least a fourth point of attachment.

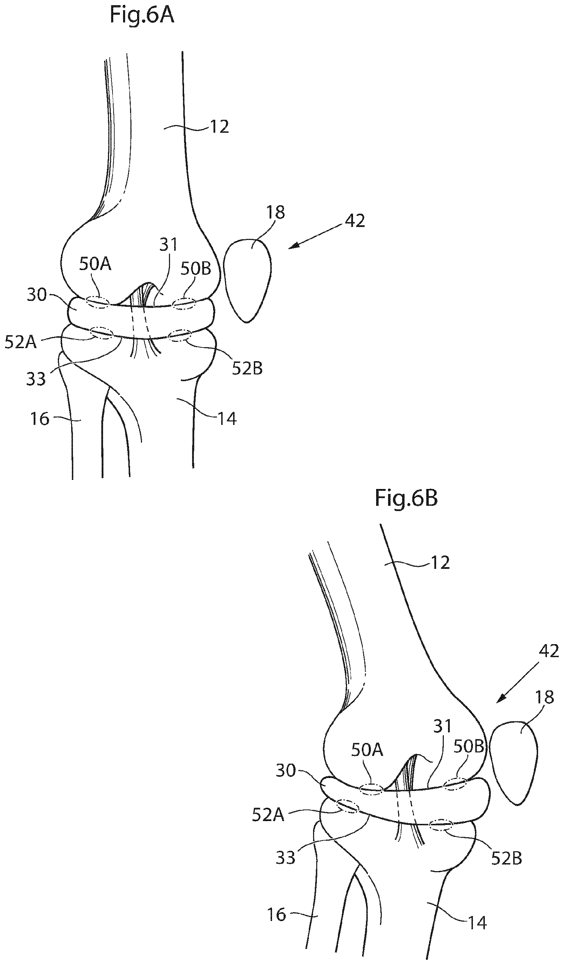

As shown in FIGURES 6A, the cushion 30 is attached by suitable means, such as by fixating sections in the form of a medical surgical adhesive or fasteners, to the femur 14 at the interface 31 of the top side of the cushion 30 and the bottom of the femur 14 at connection points 50A and 50B. Similarly, the cushion 30 is also attached by suitable means to the tibia 12 at the interface 33 of the bottom side of the cushion 30 and the top side 40 of the tibia 12 at second connection points 52A and 52B. As such, as the femur 14 moves relative to the tibia 12 when the patient bends his or her knee joint 10, as shown in FIGURE 6B, the positioning of the connection points 50A/50B and 52A/52B on the femur 14 and the tibia 12, respectively, is changed so that the connection points 50A and 50B shift relative to the connection points 52A and 52B, respectively, to accommodate a change in the orientation between the femur 14 and the tibia 12 caused by the bending of the patient's knee joint 10. This shift in the relative positioning of the attachment points 50A and 50B and 52A and 52B eliminates the sliding, and thus wear and tear, that occurs in conventional knee joint replacements, that that shown in FIGURE 2 where an upper metal piece 13 of the upper knee joint slides relative to a lower plastic piece 23 of the joint as the joint is flexed.

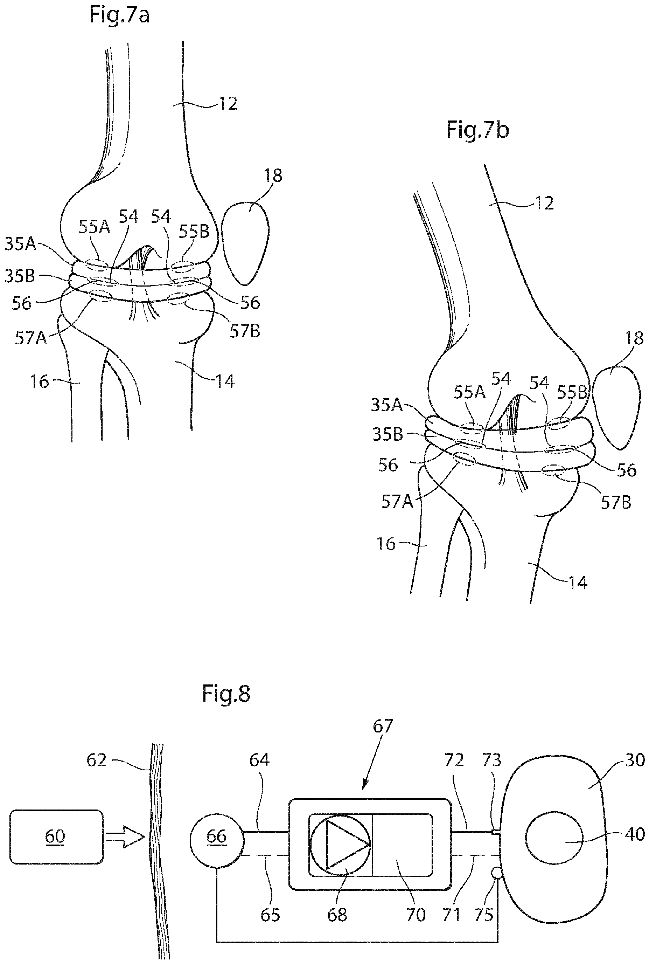

In an alternative embodiment shown in FIGURE 7A, 7B, two cushions 35A and 35B can be inserted between a patient's femur and tibia. In this arrangement, the construction of each of the two cushions would be similar to that of the cushions described above and shown in FIGURES 4A to 4D. However, the upper cushion 35A would be attached on its upper side to the patient's femur 14, while the other, lower cushion 35B would be attached on its lower side to the patient's tibia 12, again, by fixating sections in the form of a suitable medical surgical adhesive or suitable medical surgical fasteners. Positioned between the two cushions are a pair of flat metal plates 54 and 56 that, in one embodiment, would slide relative to one another as a patient would flex his or her knee so that the orientation of the femur relative to the tibia would change. In this embodiment, the upper cushion 35A would be attached on its lower side to the upper metal plate 54, while the lower cushion 35B would be attached on its upper side to the lower metal plate 56. In a further alternative embodiment, the two metal plates 54 and 56 would be fixed relative to one another so that flexing of a patient's joint would result in shifting of the fluid within each of the two cushions 35A and 35 B, so as to accommodate a change in the orientation of the patient's femur relative to the patient's tibia as the patient's knee joint is flexed. FIGURE 7B shows the embodiment of FIGURE 7A when flexed.

The cushion of the present invention can also be constructed to allow pressure to be added to or removed from the fluid filling the cushion to thereby change the level of support of a patient's body weight provided by the cushion. As shown in FIGURES 4D, the embodiment 30C of the cushion includes a valve arrangement 73 for adding fluid to or removing fluid from the cushion 30C and a pressure sensor 75 for determining the level of pressure in the cushion. Where the cushion includes such a valve arrangement, a suitable pump and reservoir of fluid are implanted in the patient's leg to allow the fluid in the cushion to be adjusted and thereby change the pressure of the fluid within the cushion.

FIGURE 8 depicts one embodiment of an apparatus 67 for performing the function of adding fluid to or removing fluid from the cushion 30C to thereby change the pressure in cushion 30C, whereby the cushion provides greater or lesser buffering between the knee joint bones 14 and 12. An implanted internal control unit 66 controls the pressure in the cushion 30C via control line 65. An external control unit 60, shown to the left of skin 62 in FIGURE 8, includes an external source of energy and a wireless remote control transmitting a control signal generated by the external source of energy. The control signal is received by a signal receiver incorporated in the implanted control unit 66, whereby the control unit 66 controls the pressure in the cushion 30C in response to the control signal. The implanted control unit 66 also uses energy from the control signal that is transmitted from external control unit 60 for operating, via a power supply line 64, the assembly 67 that includes a motor/pump unit 68 and a fluid reservoir 70, shown in FIGURE 8. In this case, the cushion 30C is hydraulically operated, i.e., hydraulic fluid is pumped by the motor/pump unit 68 from the reservoir 70 through a conduit 72 and valve arrangement 73 to the cushion 30C to increase the pressure of the fluid 32 in the cushion 30C. Conversely, hydraulic fluid is pumped by the motor/pump unit 68 back from the cushion 30C to the reservoir 70, again through a conduit 72 and valve arrangement 73, to reduce the amount of fluid 32 and thus the pressure in the cushion 30C. The pressure is measured by pressure sensor 75, which is connected to control unit 66. The external control unit 60 releases energy carried by a wireless signal and the implanted control unit 66 transforms the wireless energy into a current, for example, for powering the motor/pump unit 68 via electric power supply line 64. The implanted control unit 66 controls the motor/pump unit 68 and the pressure in the cushion 30 via control lines 65 and 71.

The fluid supply could also, in it's easiest embodiment, only comprise an injection port that is preferably implanted subcutaneously. One embodiment of an injection port is disclosed in patent application publication number US 2004/0064110 A1, the entire contents of which are incorporated by reference in this application. It should be noted that other injection port embodiments could be used with the cushion of the present invention.

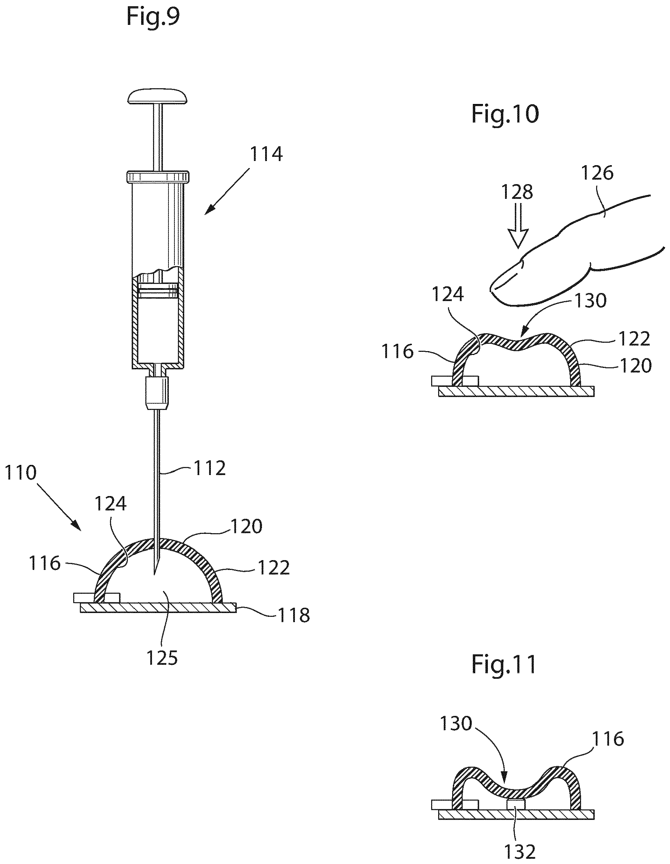

Referring to the injection port embodiment disclosed in the referenced application publication, FIGURE 9 shows an injection port 110 where a needle 112 of a syringe 114 has been injected through a membrane 116 attached to a rigid base member 18 of the injection port 110. According to the embodiment shown in FIGURE 9, membrane 116 has a semi-spherical shape in its initial, "non-depressed" position, as shown in FIGURE 9. In the embodiment shown in FIGURES 9-11, membrane 116 is comprised of three layers attached to each other: a first hard layer 120 having preferably a hardness of more than 120 Shore; a second soft central layer 122 having a hardness of less than 20 Shore; and a third hard layer 124, having a hardness suitably more than 20 Shore, but preferably about 60 Shore or more. Membrane 116 and base member 118 define a chamber 125 for fluid. However, in the most general embodiment of the injection port of the present invention, it is sufficient if membrane 116 comprises two layers, i.e., one first hard layer and one second soft layer between chamber 125 and first layer 120. First layer 120 has better strength properties than second layer 122, and second layer 122 has better sealing properties than first layer 120. Membrane 116's layers are suitably made of plastic or silicone, and preferably of silicone. Suitable silicon materials are manufactured by "Applied Silicone, Inc."

FIGURES 10 and 11 show injection port 110 of FIGURE 9 working as a hand driven pump. By using a core for membrane 116 that is very soft, i.e., elastic silicone material of less than 20 shore, it is possible to create a thinner and more elastic membrane that could be pumped by hand and still not cause leakage when a needle 112 of a syringe 114 penetrates the membrane. FIGURE 10 illustrates a finger 126 pushing (actuated by one push) membrane 116 in a direction 128 from above. Membrane 116 will then be substantially flattened, such that the surface that is faced against the finger 126 will assume a somewhat concave bowl-shape 130. Membrane 116 is then moved to a lowest position, as shown in FIGURE 11, where it is held by a locking device 132 until it is manually pressed again. When membrane 116 is actuated again, by a second push by the finger 126, the locking device 132 (which functions similar to the locking mechanism for a ballpoint pen) releases membrane 116, whereby membrane 116 is able to return to its regular convex-shaped condition as shown in FIGURE 9.

According to the method of using injection port 110 to add fluid to cushion 130, after injection port 110 is subcutaneously implanted in the patient, displaceable injection membrane 116 is used to pump fluid in fluid chamber 125 to cushion 130, which is hydraulically connected to injection port 110. The amount of fluid in fluid chamber 125 capable of being pumped to restriction device 141 using injection port 110 is calibrated by penetrating the patient's skin and membrane 116 of injection port 110 with injection needle 112 of syringe 114 to add or withdraw fluid from chamber 125. Membrane 116 is manually displaced from time to time to pump the fluid from chamber 125 of injection port 110 to implant 141 to operate the implant.

While the invention has been described in connection with what is presently considered to be the most practical and preferred embodiment, it is to be understood that the invention is not to be limited to the disclosed embodiment, but on the contrary, is intended to cover various modifications and equivalent arrangements included within the spirit and scope of the appended claims.

### LIST OF EXAMPLES OF THE INVENTIVE CONCEPT.

Example 1. A cushion adapted to be implanted in a patient's knee joint between the patient's femur and tibia bones, the cushion comprising:
   a pocket made from a flexible material, and
   a fluid filling the interior of the pocket,
   the pocket being sized to fit in the space between the patient's femur and tibia bones.
Example 2. The cushion of example 1, wherein the pocket is adapted to accommodate two interior crucible ligaments extending between the patient's femur and tibia bones.
Example 3. The cushion of example 1, wherein the cushion substantially has a size corresponding to the menisci originally in the patient's knee joint between the patient's femur and tibia bones.
Example 4. The cushion of example 1, wherein the fluid is a substantially incompressible fluid.
Example 5. The cushion of example 4, wherein the substantially incompressible fluid is water.
Example 6. The cushion of example 1, wherein the fluid is a foam.
Example 7. The cushion of example 1, wherein the fluid is a gel.
Example 8. The cushion of example 1, comprising an opening adapted to accommodate two interior crucible ligaments, wherein said opening is placed substantially in the center of the cushion through which the ligaments pass.
EXAMPLE 9. The cushion of example 1, comprising a narrow section adapted to accommodate two interior crucible ligaments in a narrow center section of the cushion, that passes between the ligaments, so that the cushion is shaped substantially like a bow tie.
Example 10. The cushion of example 1, wherein the flexible material is a polymer type of material.
Example 11. The cushion of example 1, wherein the cushion is comprised of a plurality of interior pockets filled with the fluid and isolated from another so that a rupture of one pocket results in the fluid draining from the ruptured pocket and not from the other pockets comprising the plurality of pockets.
Example 12. The cushion of example 1, wherein the cushion has a first fixating section for attaching the cushion to the bottom of the femur at at least a first point of attachment and a second fixating section for attaching the cushion to the top of the tibia at at least a second point of attachment so that, as the femur moves relative to the tibia when a patient bends his or her knee joint, the positioning of the first and second points of attachment are caused to shift relative to one another to accommodate a change in orientation between the femur and the tibia caused by the bending of the patient's knee joint.
Example 13. The cushion of example 1, wherein the cushion further comprises a valve arrangement for adding and removing fluid from the cushion to thereby change the level of pressure in the cushion and thereby the support provided by the cushion with respect to the patient's knee bones.
Example 14. The cushion of example 13, further comprising an apparatus adapted to be implanted in a patient that is connected to the valve arrangement for adding fluid to, or removing fluid from, the cushion.
Example 15. The cushion of example 14, wherein the apparatus is comprised of:
   an internal control unit adapted to be implanted in the patient,
   a fluid reservoir adapted to be implanted in the patient from which fluid is obtained or to which fluid is added for changing the level of fluid in the cushion, and
   a motor pump unit adapted to be implanted in a patient for transferring fluid from the fluid reservoir to the cushion and from the cushion to the fluid reservoir to thereby change the pressure in the cushion.
Example 16. The cushion of example 1, further comprising a second cushion positioned between the cushion and either the femur or the tibia bones so that two cushions are positioned between said bones, wherein the cushion has a first fixating section for attaching the cushion to the bottom of the femur at at least a first point of attachment and the second cushion has a second fixating section for attaching the second cushion to the top of the tibia at at least a second point of attachment.
Example 17. The cushion of example 16, further comprising first and second flat metal plates positioned between the cushion and the second cushion, the first and second metal plates being attached to the cushion and the second cushion so that the plates slide relative to one another as the orientation between the femur and the tibia changes as the patient bends his or her knee.
Example 18. The cushion of example 17, wherein the first and second metal plates are fixed relative to one another so that the first and second points of attachment are caused to shift relative to one another to accommodate a change in orientation between the femur and the tibia caused by the bending of the patient's knee joint.
Example 19. The cushion of example 13, further comprising a pressure sensor connected to the cushion that determines the level of pressure in the cushion.
Example 20. The cushion of example 15, further comprising an external control unit including an external source of energy and a wireless remote control transmitting to the internal control unit a control signal generated by the external source of energy.
Example 21. The cushion of example 15, further comprising a pressure sensor connected to the cushion that determines the level of pressure in the cushion and to the internal control unit for providing a signal representative of the level of pressure in the cushion.
Example 22. The cushion of example 21, wherein the apparatus adds fluid to, or removes fluid from, the cushion, depending on the level of pressure in the cushion measured by the pressure sensor connected to the cushion.
Example 23. The cushion of example 12, wherein the first and second fixating sections comprise a surgical adhesive and/or at least one surgical fastener.
Example 24. The cushion of example 16, wherein the first and second fixating sections comprise a surgical adhesive and/or at least one surgical fastener.
Example 25. The cushion of example 13, further comprising an implanted injection port for adding fluid to, or removing fluid from, the cushion.
Example 26. A system adapted to be implanted in a patient's knee joint, the knee joint comprising a prolonged femoral length axis, being the prolongation of the length axis of the femoral bone which passes the knee joint and continues substantially to the tibia length axis, when the leg is in its fully extended state, wherein:
   said system comprises a first cushion according to example 1, and
   a second cushion according to example 1, wherein:
      said first cushion is adapted to mainly be positioned on the medial side of said femoral length axis, and wherein
      said second cushion is adapted to mainly be positioned on the lateral side of said femoral length axis, such that:
         said first cushion is adapted to mainly carry a load originating from the weight of the patient distributed through the medial condyle, and
         said second cushion is adapted to mainly carry a load originating from the weight of the patient distributed through the lateral condyle, when said first and second cushions are implanted.
Example 27. The system of example 26, further comprising a third cushion positioned between the first cushion placed at the medial condyle and either at the femur or the tibia bones so that two cushions are positioned between the bones, wherein said first cushion has a first fixating section for attaching the cushion to the bottom of the femur at, at least a first point of attachment, and the third cushion has a third fixating section for attaching the third cushion to the top of the tibia at, at least a third point of attachment,
   wherein the system further comprising a fourth cushion positioned between the second cushion and either the femur or the tibia bones at the lateral condyle so that two cushions are positioned between said bones, wherein the second cushion has a second fixating section for attaching the cushion to the bottom of the femur at, at least a second point of attachment and the fourth cushion has a fourth fixating section for attaching the fourth cushion to the top of the tibia at, at least a fourth point of attachment.
Example 28. The cushion of example 1, wherein the flexible pocket is adapted to at least partly be attached to the tibia and femoral bones, wherein the flexible fluid filled pocket is adapted to allow a friction free movement between the tibia and femur, said movement instead based on material flexibility and fluid movement
Example 29. The cushion of example 28, wherein the cushion has a first fixating section for attaching the cushion to the bottom of the femur at, at least a first point of attachment and a second fixating section for attaching the cushion to the top of the tibia at at least a second point of attachment so that, as the femur moves relative to the tibia when a patient bends his or her knee joint, the positioning of the first and second points of attachment are caused to shift relative to one another to accommodate a change in orientation between the femur and the tibia caused by the bending of the patient's knee joint.
Example 30. The cushion of example 16, wherein the flexible pocket in both the first and second cushion is adapted to at least partly be attached to the tibia and femoral bones, wherein the flexible fluid filled pocket is adapted to allow friction free movement between the tibia and femur, said movement instead based on material flexibility and fluid movement,
   said cushion further comprising a first and second flat plate positioned between the cushion and the second cushion, the first and second plates being attached to the cushion and the second cushion so that the plates slide relative to one another as the orientation between the femur and the tibia changes as the patient bends his or her knee, wherein said plates is adapted to be placed parallel to each other for allowing a larger weight carrying surface.
Example 31. A method for implanting a cushion in a patient's knee joint between the patient's femur and tibia bones, the method comprising the steps of:
   - placing a pocket made from a flexible material, with a fluid filling the interior of the pocket,
   - fitting in the space between the patient's femur and tibia bones.

## Claims

1. A cushion adapted to be implanted in a patient's knee joint between the patient's femur and tibia bones, the cushion comprising:
a pocket made from a flexible material, and
a fluid filling the interior of the pocket,
the pocket being sized to fit in the space between the patient's femur and tibia bones, **characterized in that**
the cushion has a first fixating section for attaching the cushion to the bottom of the femur at at least a first point of attachment and a second fixating section for attaching the cushion to the top of the tibia at at least a second point of attachment so that, as the femur moves relative to the tibia when a patient bends his or her knee joint, the positioning of the first and second points of attachment are caused to shift relative to one another to accommodate a change in orientation between the femur and the tibia caused by the bending of the patient's knee joint.

2. The cushion of claim 1, wherein the pocket is adapted to accommodate two interior crucible ligaments extending between the patient's femur and tibia bones.

3. The cushion of claim 1, wherein the cushion substantially has a size corresponding to the menisci originally in the patient's knee joint between the patient's femur and tibia bones.

4. The cushion of claim 1, wherein the fluid is a substantially incompressible fluid.

5. The cushion of claim 4, wherein the substantially incompressible fluid is water.

6. The cushion of claim 1, wherein the fluid is a foam.

7. The cushion of claim 1, wherein the fluid is a gel.

8. The cushion of claim 1, comprising an opening adapted to accommodate two interior crucible ligaments, wherein said opening is placed substantially in the center of the cushion through which the ligaments pass.

9. The cushion of claim 1, comprising a narrow section adapted to accommodate two interior crucible ligaments in a narrow center section of the cushion, that passes between the ligaments, so that the cushion is shaped substantially like a bow tie.

10. The cushion of claim 1, wherein the flexible material is a polymer type of material.

11. The cushion of claim 1, wherein the cushion is comprised of a plurality of interior pockets filled with the fluid and isolated from another so that a rupture of one pocket results in the fluid draining from the ruptured pocket and not from the other pockets comprising the plurality of pockets.

12. The cushion of claim 1, wherein the cushion further comprises a valve arrangement for adding and removing fluid from the cushion to thereby change the level of pressure in the cushion and thereby the support provided by the cushion with respect to the patient's knee bones.

13. The cushion of claim 12, further comprising an apparatus adapted to be implanted in a patient that is connected to the valve arrangement for adding fluid to, or removing fluid from, the cushion.

14. The cushion of claim 13, wherein the apparatus comprises:
an internal control unit adapted to be implanted in the patient,
a fluid reservoir adapted to be implanted in the patient from which fluid is obtained or to which fluid is added for changing the level of fluid in the cushion, and
a motor pump unit adapted to be implanted in a patient for transferring fluid from the fluid reservoir to the cushion and from the cushion to the fluid reservoir to thereby change the pressure in the cushion.

15. A system adapted to be implanted in a patient's knee joint, the knee joint comprising a prolonged femoral length axis, being the prolongation of the length axis of the femoral bone which passes the knee joint and continues substantially to the tibia length axis, when the leg is in its fully extended state, wherein:
said system comprises a first cushion according to claim 1, and
a second cushion according to claim 1, wherein:
said first cushion is adapted to mainly be positioned on the medial side of said femoral length axis, and wherein
said second cushion is adapted to mainly be positioned on the lateral side of said femoral length axis, such that:
said first cushion is adapted to mainly carry a load originating from the weight of the patient distributed through the medial condyle, and
said second cushion is adapted to mainly carry a load originating from the weight of the patient distributed through the lateral condyle, when said first and second cushions are implanted.e
